Europäisches Patentamt

(19) European Patent Office

Office européen des brevets

(11) Publication number: **0 348 019 B1**

# (12) EUROPEAN PATENT SPECIFICATION

(45) Date of publication of patent specification :
18.12.91 Bulletin 91/51

(51) Int. Cl.⁵ : **A61M 16/18**

(21) Application number : **89301245.0**

(22) Date of filing : **09.02.89**

(54) Anaesthetic vaporiser.

(30) Priority : **27.04.88 GB 8809896**

(43) Date of publication of application :
**27.12.89 Bulletin 89/52**

(45) Publication of the grant of the patent :
**18.12.91 Bulletin 91/51**

(84) Designated Contracting States :
**DE GB NL**

(56) References cited :
**GB-A- 1 224 478
GB-A- 2 084 469
US-A- 3 575 168**

(73) Proprietor : **The BOC Group plc
Chertsey Road
Windlesham Surrey GU20 6HJ (GB)**

(72) Inventor : **Gregory, Raymond Stanley
1 Langley Grove
Bingley Yorkshire (GB)**

(74) Representative : **Belcher, Simon James et al
Urquhart-Dykes & Lord Tower House Merrion
Way
Leeds LS2 8PA (GB)**

Jouve, 18, rue Saint-Denis, 75001 PARIS

## Description

The present invention relates to anaesthetic vaporisers.

In UK Patent No. 1,385,670, there is described a gas administration apparatus including a back bar onto which one or more anaesthetic vaporisers of the bypass type may be mounted in a removable plug-in fashion.

When two or more vaporisers each containing a different anaesthetic agent are mounted on the same back bar it is desirable to prevent vapour from an upstream vaporiser contaminating a downstream vaporiser. Any such contamination could have a detrimental effect on a patient.

UK Patent 1,385,670 further describes the use of a selector valve which can be used to direct carrier gas from a gas mixer to a selected vaporiser. The vaporiser not so selected is totally isolated and thus cross contamination of the vaporisers is avoided.

UK Patent No. 2,052,271 and UK published Patent Application No. 2,193,642 each described an interlock system for use when two or more vaporisers are mounted on a back bar of an anaesthesia machine. The interlock systems each include extensible pins which physically prevent a control valve of more that one vaporiser being turned on at any one time.

UK Patent No. 2,052,271 also describes a mechanical system which, when a vaporiser is mounted on a back bar, opens respective valve members in ports located on the back bar only when its control valve is moved to its operating or ON position.

The interlock systems using extensible pins are relatively simple and inexpensive to produce. However, they rely on the downstream vaporisers having no internal leaks which would permit vapour to flow past a seal or valve seating.

As described in UK Patent No. 2,052,271 the interlock system using extensible pins can be coupled with a mechanical system. Although such combined systems offer a good degree of protection against cross contamination they are more complex mechanically and therefore relatively expensive.

It is an aim of the present invention to provide an anaesthetic vaporisers which is economical to manufacture, easy to service and even under fault conditions, will not permit cross contamination of a second or downstream vaporiser when both are mounted on a back bar of an anaesthesia machine.

Accordingly, the present invention provides an anaesthetic vaporiser of the by-pass type in which carrier gas is initially divided into a first stream passing through a vaporising chamber to become mixed with vapour from a volatile liquid anaesthetic and a second by-pass stream the two streams subsequently recombining, the first stream being controlled by a control valve, characterised in that at least two spaced grooves are formed in a facing surface on the control valve and are at all times in communication with atmosphere, each groove communicating with a bore on a seating face of a closure plate directly opposed to the facing surface of the control valve, said bores communicating with atmosphere.

An embodiment of the invention will now be described by way of example, reference being made to the Figures of the accompanying diagrammatic drawings in which :

Figure 1 is a sectional elevation generally on line I-I of Figure 2 but partly in diagrammatic form for ease of description of a known anaesthetic vaporiser of the by-pass type as described in UK Patent 1,224,478 ;

Figure 2 is a plan view from above of a seating face on which a facing surface of a control valve forming part of the vaporiser of Figure 1, is superimposed, also showing in broken lines the outline of the superimposed facing surface with raised areas of this shown in broken line shading;

Figure 3 is a sectional plan view from below of a facing surface of a new control valve ;

Figure 4 is an elevation of the control valve of Figure 3 and a closure plate ; and

Figure 5 is a sectional elevation generally the same as Figure 1 but illustrating a new vaporiser including the control valve and closure plate of Figure 4.

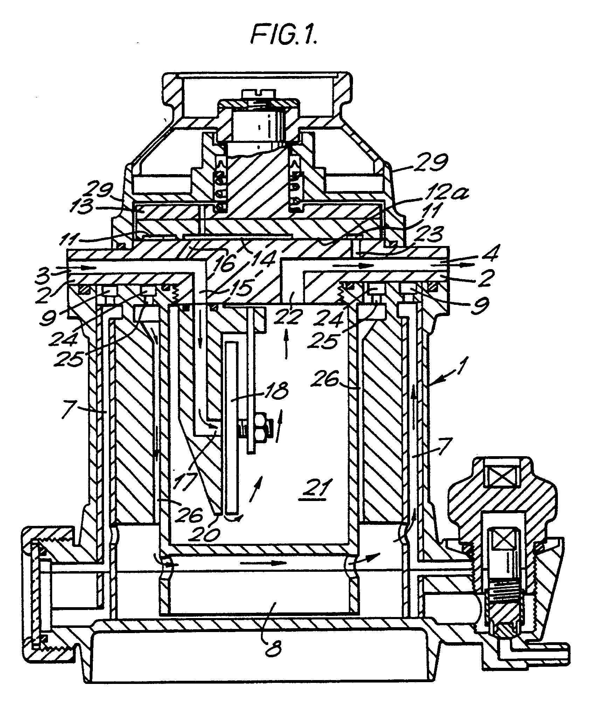

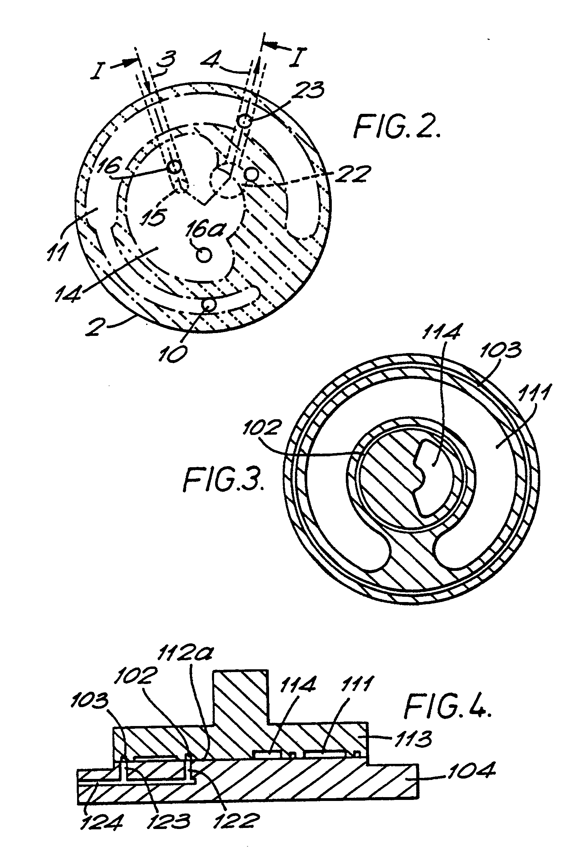

Referring first to Figures 1 and 2, these Figures illustrate a known anaesthetic vaporiser 1 of the by-pass type in which, when in the ON position, carrier gas enters an inlet 3 in a top closure plate 2 and at the entry to a port 16 is divided into two streams. A first stream passes through the port 16 and the second by-pass stream passes down a passage 15 including a port 17 controlled by a thermally responsive valve 18 which acts to increase the valve opening with increase in temperature and to reduce the valve opening with decrease in temperature. This by-pass gas stream emerges through a restricted passage 20 into a chamber 21. There is an outlet 22 from this chamber which joins an outlet 4 from the vaporiser.

The first stream passes through port 16 into a recess 14 in a facing surface 12a of a control valve 13 mounted for rotatable movement in an annular guide part 29. From the recess 14 the gas passes down a passage 16a into an annular groove 24 which communicates via a series of holes 25 with a very narrow annular passage 26. The gas flows along the passage 26 onto the surface of liquid anaesthetic 8.

Carrier gas and entrained vapour then travels up annular passage 7, passage 9 and passage 10 in the top plate 2 into a control groove 11 also formed in the facing surface 12a of the control valve 13. Carrier gas and entrained vapours then flow through passage 23 towards the outlet 4.

When the control valve 13 is moved to its OFF

2

position the passages 10, 16a are blocked by facing surface 12a so that carrier gas and vapours from an upstream vaporiser will flow from the inlet 3 down the by-pass system through the valve 18, controlled port 17 into the chamber 21 to pass up to the outlet 4. It will be clear that when an upstream vaporiser is turned ON vapour from that vaporiser will flow through the passages 15, 22 of a downstream vaporiser and will also be present in port 16. Any leakage across the facing surface 12a of control valve 13 will tend to allow vapour to cross the face of the valve and contaminate the sump region of the downstream vaporiser.

Referring also to Figures 3 to 5 an anaesthetic vaporiser 201 is similar in some respect to the vaporiser 1 and for convenience like reference numerals will denote like parts. The vaporiser 201 includes a control valve 113 which is formed on its facing surface 112a with a drug control groove 111, by-pass recess 114, a first outer vent groove 103, and a second inner vent groove 102. The grooves 102, 103 (as shown) are circular and each registers with a bore 122 and 123 respectively in a seating face of the top closure plate 104. Both bores 122, 123 communicate with a common passageway 124 which communicates with the atmosphere.

Carrier gas enters the vaporiser 201 at inlet 100 formed in the annular guide part 29 and flows via a passage 101 into the recess 114. When the vaporiser control valve 113 is in its OFF position the recess 114 connects the carrier gas directly to the outlet 4 via passage 15 through the valve 18, chamber 21 and passage 22.

Any carrier gas or vapour which attempts to leak across the facing surface is collected in groove 102 and vented to atmosphere via bore 122 and passageway 124. Any carrier gas and vapour which attempts to leak around the periphery of the control valve 113 is collected in groove 103 and again vented to atmosphere via bore 123 and passageway 124.

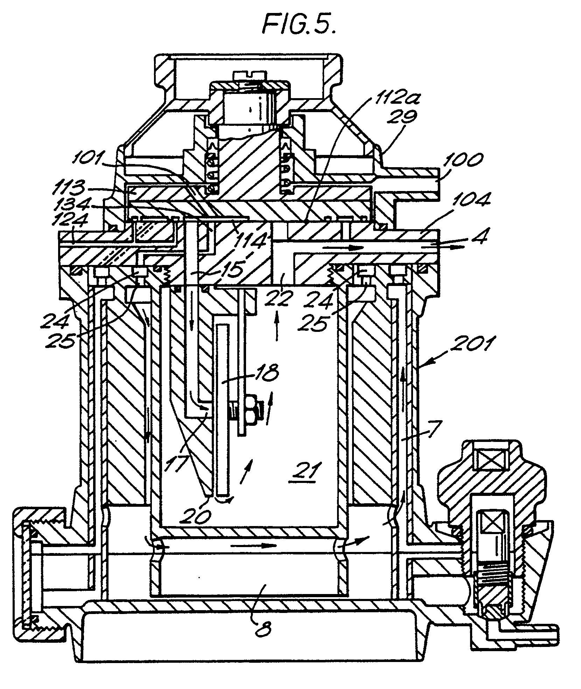

When the control valve 113 is moved to an ON position, it is rotated to the position shown in Figure 5 in which recess 114 now directs gas both into the by-pass stream, that is through the valve 18, via the passage 15 and also into the sump area via the passage 134 annular groove 24 and holes 25. Gas and anaesthetic vapour exit from the vaporiser as previously described via outlet 4.

It will be apparent that some part of the valve 113 is, at all times, in communication with the atmosphere and the grooves 102, 103 are positioned to form a barrier between any gas and vapour flow entering a vaporiser and the passages to and from the sump area of the vaporiser. Thus in the event of any trace leakage across the facing surface 112a of the control valve 113 the migrating vapour is intercepted and allowed to emerge to atmosphere via the bores 122, 123 and passageway 124.

It will be apparent also that the gas seal formed by the engagement of the facing surface 112a and the seating face of closure plate 104 could be achieved by the use of resilient sealing members without affecting the function of the venting grooves 102 and 103. Further, the grooves 102, 103 could be formed in the seating face of the closure plate 104.

## Claims

1. An anaesthetic vaporiser (201) of the by-pass type in which carrier gas is initially divided into a first stream passing through a vaporising chamber to become mixed with vapour from a volatile liquid anaesthetic (8) and a second by-pass stream, the two streams subsequently recombining, the first stream being controlled by a control valve (113), <u>characterised in that at least two spaced grooves (102, 103) are</u> formed in a facing surface (112a) on the control valve (113) and are at all times in communication with the atmosphere, each groove communicating with a bore on a seating face of a closure plate (104) directly opposed to the facing surface (112a) of the control valve (113), said bores (122, 123) communicating with atmosphere.

2. An anaesthetic vaporiser as claimed in claim 1, in which the grooves (102, 103) are in the form of spaced concentric circles.

## Patentansprüche

1. Anaestheticum-Zerstäuber (201) des By-pass-Typs, bei dem ein Trägergas zunächst geteilt wird in einen ersten Strom, der durch eine Verdampfungskammer geführt wird, wo er mit Dampf eines flüchtigen, flüssigen Anaestheticums (8) gemischt wird, und einen zweiten By-pass-Strom, wobei beide Strome schließlich wieder vereinigt werden und der erste Strom durch ein Steuerventil (113) gesteuert wird, dadurch gekennzeichnet, daß in einer Anlagefläche (112a) des Steuerventils (113) mindestens zwei voneinander getrennte Nuten (102, 103) gebildet werden und zu jeder Zeit in Verbindung mit der Atmosphäre stehen, wobei jede Nut in Verbindung steht mit einer Bohrung in einer Anlagefläche einer Verschlußplatte (104) unmittelbar gegenüber der Anlagefläche (112a) des Steuerventils (113), wobei die Bohrungen (122, 123) mit der Atmosphäre in Verbindung stehen.

2. Anaestheticum-Verdampfer nach Anspruch 1, bei dem die Nuten (102, 103) in Form von in einem Abstand befindlichen konzentrischen Kreisen ausgebildet sind.

## Revendications

1. Vaporisateur d'anesthésique (201) du type à dérivation dans lequel le gaz porteur est initialement divisé en un premier flux traversant une chambre de vaporisation pour se mélanger avec la vapeur provenant d'un anesthésique liquide volatile (8) et un second flux de dérivation, les deux flux se recombinant ultérieurement, le premier flux étant commandé par une soupape de réglage (113), caractérisé en ce qu'au moins deux rainures séparées (102, 103) sont formées dans une surface de contact (112a) sur la soupape de réglage (113) et sont tout le temps en communication avec l'air ambiant, chaque rainure communiquant avec un alésage sur une face d'appui d'une plaque de fermeture (104) directement opposée à la surface de contact (112a) de la soupape de réglage (113), lesdits alésages (122, 123) communiquant avec l'air ambiant.

2. Vaporisateur d'anesthésique selon la revendication 1, dans lequel les rainures (102, 103) sont sous la forme de cercles concentriques espacés.

FIG.1.

FIG.2.

FIG.3.

FIG.4.

## FIG.5.